(19) **Europäisches Patentamt**
**European Patent Office**
**Office européen des brevets**

(11) **EP 4 160 608 A1**

(12) **EUROPEAN PATENT APPLICATION**
published in accordance with Art. 153(4) EPC

(43) Date of publication:
**05.04.2023 Bulletin 2023/14**

(21) Application number: **21812354.5**

(22) Date of filing: **30.04.2021**

(51) International Patent Classification (IPC):
***G16H 20/13*** (2018.01)

(86) International application number:
**PCT/CN2021/091490**

(87) International publication number:
**WO 2021/238584 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2020 CN 202010479267**

(71) Applicant: **BEIJING JINGDONG TUOXIAN TECHNOLOGY CO., LTD.**
**Beijing 100176 (CN)**

(72) Inventor: **MENG, Fei**
**Beijing 100176 (CN)**

(74) Representative: **Ruuskanen, Juha-Pekka**
**Page White & Farrer Limited**
**Bedford House**
**21A John Street**
**London WC1N 2BF (GB)**

(54) **PRESCRIPTION ORDER DISTRIBUTION METHOD, SYSTEM AND DEVICE, AND STORAGE MEDIUM**

(57)     A prescription order distribution method, system and device and a storage medium, which relate to the technical field of data processing. The prescription order distribution method comprises: in response to receiving a prescription order from an end user, acquiring busyness information of a plurality of pharmacist groups (S102), wherein each pharmacist group comprises one or more pharmacists, each pharmacist group corresponds to one queue, and pharmacists in the pharmacist group review prescription orders in a corresponding queue; according to the busyness information, determining a pharmacist group that reviews the prescription order (S104); sending the prescription order to a queue corresponding to the pharmacist group reviewing the prescription order (S106); and acquiring a review result of the prescription order by the pharmacists in the pharmacist group (S108). Since the processing conditions of the plurality of pharmacists in the pharmacist group are comprehensively considered, the processing capabilities of individual pharmacists will not overly affect the overall processing capabilities of the pharmacist group, so that prescription orders may be promptly processed. Therefore, the processing efficiency of orders may be improved in a prescription order distribution scenario.

FIG. 1

EP 4 160 608 A1

# Description

## CROSS-REFERENCE TO RELATED APPLICATIONS

This application is based on the application with a CN application number of 202010479267.9 and the filing date being on May 29, 2020, and claims its priority. The disclosure of this CN application as a whole is incorporated into the present application herein by reference.

## TECHNICAL FIELD

The present disclosure relates to the technical field of data processing, and in particular, to a prescription order distribution method, system, device and storage medium.

## BACKGROUND

Prescriptions are patient medication credentials which are medical documents issued by registered licensed physicians and licensed assistant physicians for patients during diagnosis and treatment activities, and reviewed, dispensed, and checked by professional pharmacy technicians (hereinafter referred to as pharmacists) who have obtained professional pharmacy professional qualifications. With the development of Internet technology, in the medical and pharmaceutical industry, it is gradually popular to provide services to people through the Internet. In an offline hospital, a patient's prescription order needs to be reviewed by a pharmacist, and an ordered prescription drug is issued for a patient only if the prescription can pass the review of the pharmacist. This step is also required in the Internet hospital scenario to improve the safety of medication. For example, a prescription order can be reviewed by a system-assigned pharmacist. During activities such as consultations of special order, speed consultations, e-commerce shopping festivals, and merchant promotions, the surge in the number of prescription orders requires the system to quickly complete the distribution and return the review results as soon as possible.

In non-pharmaceutical fields, some order distribution processes have emerged in some businesses, such as the online car-hailing service. Common order distribution schemes are as follows:

(1) Homogeneous distribution: by traversing order executors, orders are evenly distributed to the executors;

(2) Order grabbing: executors get orders through order grabbing;

(3) Distribution orders according to the rule of proximity: give priority to nearby executors when distributing orders;

(4) Distribution orders according to customer level: give priority to high-level customers when distributing orders;

(5) Distribution orders according to the rule of global optimization: a global optimal distribution scheme is developed according to certain rules taking factors such as the user's gender, vehicle use time, and destination into consideration.

## SUMMARY

According to a first aspect of some embodiments of the present invention, a method for prescription order distribution is provided, comprising: acquiring busyness degree information of a plurality of pharmacist groups in response to receiving a prescription order from a user terminal, wherein each of the pharmacist groups corresponds to a queue and comprises pharmacist(s) to review prescription order(s) in the corresponding queue; determining a pharmacist group to review the prescription order according to the busyness degree information; sending the prescription order to a queue corresponding to the pharmacist group to review the prescription order; and acquiring a review result of the prescription order given by a pharmacist in the pharmacist group.

In some embodiments, acquiring busyness degree information of a plurality of pharmacist groups comprises: acquiring busyness degree information of a plurality of pharmacist groups from a cache in response to receiving a prescription order from the user terminal, wherein the busyness degree information in the cache is retrieved from a database and stored in the cache.

In some embodiments, the busyness degree information in the cache has a data lock.

In some embodiments, the distribution method of prescription order further comprises: modifying the busyness degree information of the corresponding pharmacist group in the database in response to the prescription order being sent to the queue corresponding to the pharmacist group to review the prescription order; and modifying the busyness degree information of the corresponding pharmacist group in the database, and storing the review result of the prescription order in the database in response to completing the review of the prescription order by the pharmacist in the pharmacist group.

In some embodiments, the database is an HBase database, and the distribution method further comprises: synchronizing the modified busyness degree information in the HBase database to the cache and a persistence database; and synchronizing the review result of the prescription order in the HBase database to the persistence database.

In some embodiments, the busyness degree information comprises information indicating whether a pharmacist group is idle and a current processing capacity value of the pharmacist group, and determining a pharmacist group to review the prescription order according to the busyness degree information comprises: determining an idle pharmacist group as the pharmacist group to review the prescription order in case that there is currently an idle pharmacist group; and determining a pharmacist group with a greatest current processing capacity value as the pharmacist group to review the prescription order in case that there is currently no idle pharmacist group.

In some embodiments, the idle pharmacist group is a pharmacist group having an idle value greater than a preset value, wherein the idle value is a difference between a sum of actual amount of processing of all pharmacist(s) in the pharmacist group and a number of prescription order(s) in a queue corresponding to the pharmacist group; and the current processing capacity value is a difference between a sum of actual amount of processing of online pharmacist(s) in a pharmacist group and a number of prescription order(s) in a queue corresponding to the pharmacist group.

In some embodiments, the actual amount of processing of each pharmacist is a product of a historical average amount of processing in unit time and an online time ratio of the pharmacist.

In some embodiments, the distribution method of prescription order further comprises: monitoring an online status of pharmacists in each pharmacist group; and for a pharmacist group without any online pharmacist, sending prescription order(s) in a queue corresponding to this pharmacist group to a queue of another pharmacist group.

In some embodiments, the another pharmacist group is a pharmacist group with a greatest current processing capacity value.

In some embodiments, the distribution method of prescription order further comprises: determining an information import threshold according to the busyness degree information of the plurality of pharmacist groups; sending review result(s) of prescription order(s) given by pharmacists in the pharmacist groups to an import message queue and a backup storage device, wherein the review result (s) in the import message queue are sequentially read and stored to the database; and instructing the backup storage device to send the stored review result(s) to the database for storage in response to monitoring that a number of message(s) in the import message queue is greater than the information import threshold.

According to a second aspect of some embodiments of the present invention, a distribution system of prescription order is provided, comprising: a plurality of queues, each queue corresponding to a pharmacist group, and each pharmacist group comprising one or more pharmacists to review prescription orders in the corresponding queue; a prescription processing node for acquiring busyness degree information of a plurality of pharmacist groups in response to receiving a prescription order from a user terminal; determining a pharmacist group to review the prescription order according to the busyness degree information; and sending the prescription order to a queue corresponding to the pharmacist group to review the prescription order; and a review result acquisition node for acquiring a review result of the prescription order given by a pharmacist in the pharmacist group.

In some embodiments, the system for prescription order distribution further includes: a cache for storing busyness degree information of the plurality of pharmacist groups, wherein the busyness degree information in the cache is retrieved from a database and stored in the cache.

In some embodiments, the busyness degree information in the cache has a data lock.

In some embodiments, the distribution system of prescription order further comprises: a persistence database for storing the busyness degree information and the review result of the prescription order; and an HBase database for storing busyness degree information synchronized with the persistence database, wherein the busyness degree information in the HBase database is further synchronized to the cache.

In some embodiments, the distribution system of prescription order further comprises: a storage processing node for: modifying the busyness degree information of the corresponding pharmacist group in a database in response to the prescription order being sent to the queue corresponding to the pharmacist group to review the prescription order; and modifying the busyness degree information of the corresponding pharmacist group in the database, and storing the review result of the prescription order in the database in response to completing the review of the prescription order by the pharmacist in the pharmacist group.

In some embodiments, the storage processing node is further configured for: determining an information import threshold according to the busyness degree information of the plurality of pharmacist groups; sending review result(s) of prescription order(s) given by pharmacists in the pharmacist groups to an import message queue and a backup storage device, wherein the review result(s) in the import message queue are sequentially read and stored to the database; and instructing the backup storage device to send the stored review result(s) to the database for storage in response to monitoring that a number of message(s) in the import message queue is greater than the information import threshold.

In some embodiments, a prescription processing node comprises a plurality of distributed sub-nodes.

According to a third aspect of some embodiments of the present invention, a device for prescription order distribution is provided, including: a memory; a processor coupled to the memory, the processor configured to, based on instructions stored in the memory, carry out any one of the foregoing prescription order distribution methods.

According to a fourth aspect of some embodiments of the present invention, a non-transitory computer-readable storage medium is provided on which a computer program is stored, which executed by a processor, implement any one of the foregoing prescription order distribution methods.

Some embodiments of the present invention have the following advantages or beneficial effects: The embodiment of the present invention performs order distribution in units of pharmacist groups. After the prescription order is distributed to a queue, a pharmacist who is in charge of processing the order can be determined according to the situation of each pharmacist in the pharmacist group. Since the processing conditions of multiple pharmacists in the pharmacist group are comprehensively considered, the processing ability of individual pharmacists will not immoderately affect the overall processing ability of the pharmacist group, so that prescription orders can be processed timely. Therefore, the above embodiment can improve order processing efficiency in the scenario of distribution prescription orders. The above method is applicable even in high concurrency scenarios.

Other features and advantages of the present invention will become apparent from the following detailed description of exemplary embodiments of the present invention with reference to the accompanying drawings.

## BRIEF DESCRIPTION OF THE DRAWINGS

In order to more clearly explain the embodiments of the present invention or the technical solutions in the prior art, a brief introduction will be given below for the drawings required to be used in the description of the embodiments or the prior art. It is obvious that, the drawings illustrated as follows are merely some of the embodiments of the present invention. For a person skilled in the art, he or she may also acquire other drawings according to such drawings on the premise that no inventive effort is involved.

　　FIG. 1 shows a schematic flowchart of a distribution method of prescription order according to some embodiments of the present invention;
　　FIG. 2 shows a schematic flowchart of a method for determining a pharmacist group according to some embodiments of the present invention;
　　FIG. 3 shows a schematic flowchart of a disaster recovery method according to some embodiments of the present invention;
　　FIG. 4 shows a schematic flowchart of an MQ compensation method according to some embodiments of the present invention;
　　FIG. 5 shows a schematic structural diagram of a distribution system of prescription order according to some embodiments of the present invention;
　　FIG. 6 shows a schematic structural diagram of a distribution device of prescription order according to some embodiments of the present invention;
　　FIG. 7 shows a schematic structural diagram of a distribution device of prescription order according to other embodiments of the present invention.

## DETAILED DESCRIPTION

Below, a clear and complete description will be given for the technical solution of an embodiment of this invention with reference to the figures. Obviously, merely some embodiments of this invention, rather than all embodiments thereof, is given herein. The following description of at least one exemplary embodiment is in fact merely illustrative and is in no way intended as a limitation to the invention, its application or use. All other embodiments obtained by those of ordinary skill in the art based on the embodiments of the present disclosure without creative efforts shall fall within the protection scope of the present disclosure.

Unless otherwise specified, the relative arrangement, numerical expressions and numerical values of the components and steps set forth in these examples do not limit the scope of the invention.

At the same time, it should be understood that, for ease of description, the dimensions of the various parts shown in the drawings are not drawn to actual proportions.

Techniques, methods, and apparatus known to those of ordinary skill in the relevant art may not be discussed in detail, but where appropriate, these techniques, methods, and apparatuses should be considered as part of the specification.

Of all the examples shown and discussed herein, any specific value should be construed as merely illustrative and not as a limitation. Thus, other examples of exemplary embodiments may have different values.

Notice that, similar reference numerals and letters are denoted by the like in the accompanying drawings, and therefore, once an article is defined in a drawing, there is no need for further discussion in the accompanying drawings.

After analysis, the inventors found that pharmacists need to review prescription orders according to their professional knowledge, and the orders can be processed only after some review results are obtained. Therefore, in the scenario of distributing prescription orders, the average response time of the server has a strong dependence on the work status of the pharmacists. For example, the average processing time of different pharmacists and whether the pharmacists are good at the fields of prescription orders will affect the processing time of the prescription orders. Unlike other products on e-commerce platforms, users need to get the review results as soon as possible to determine whether they can buy desired medicines.

However, in the scenario of online car hailing and distribution, the order executors, that is, the drivers can complete the processing of the orders by directly selecting whether to accept the orders. Therefore, the processing capability of the order executors is not considered in the related techniques. Moreover, those factors such as customer priority, distance to user, gender that are taken into account in the related techniques are not applicable to the prescription order distribution scenario.

Therefore, the related techniques cannot provide a solution that can improve the efficiency of order processing and is suitable for the scenario of distributing prescription orders.

A technical problem to be solved by the embodiments of the present invention is: how to provide a solution that can improve order processing efficiency and is suitable for the scenario of distributing prescription orders.

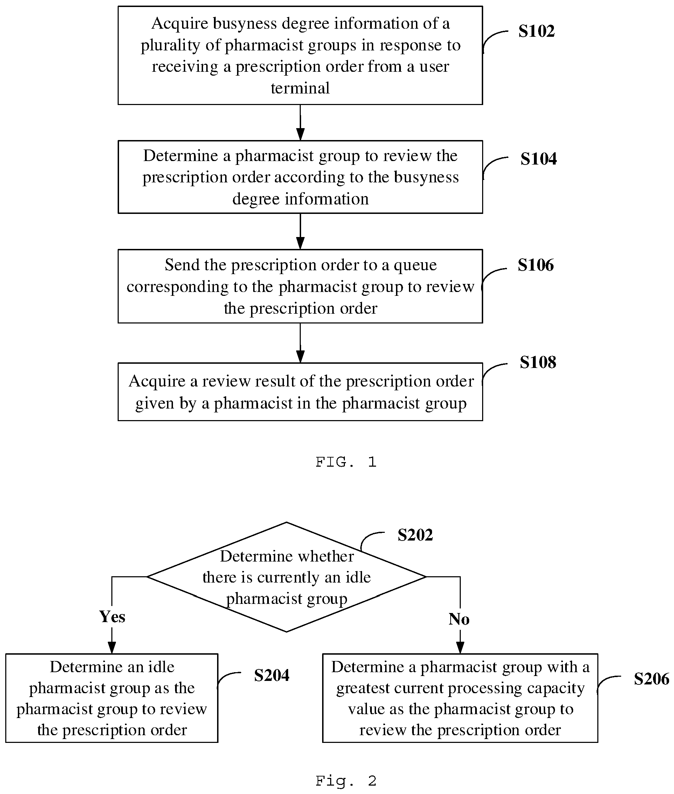

FIG. 1 shows a schematic flowchart of a distribution method of prescription order according to some embodiments of the present invention. As shown in FIG. 1, the distribution method of prescription order of this embodiment comprises steps S102 to S108.

In step S102, busyness degree information of a plurality of pharmacist groups is acquired in response to receiving a prescription order from a user terminal, wherein each of the pharmacist groups corresponds to a queue and comprises pharmacist(s) to review prescription order(s) in the corresponding queue, these queues constituting an asynchronous queue group. The queue corresponding to each pharmacist group is, for example, a cache queue, so as to speed up reading prescription orders by a prescription order processing device of a pharmacist.

In some embodiments, a user sends a drug purchase request through an application in his terminal. After receiving the request, an application server generates a prescription order. In some embodiments, after a user communicates with an online Internet doctor, the Internet doctor prescribes a prescription and generates a prescription order.

In some embodiments, pharmacists are grouped according to their processing capabilities: pharmacists are sorted according to their processing capabilities, and those pharmacists whose distance in the queue in the sorting result is a preset value are grouped. For example, among 100 pharmacists, the 1st, 11th, 21st... pharmacists are grouped, and the 2nd, 12th, 22nd... pharmacists are grouped. Thus, the overall processing capacities of the different pharmacist groups are close to each other. Pharmacists can also be grouped in other ways as needed.

In step S104, a pharmacist group to review the prescription order is determined according to the busyness degree information. For example, the prescription order can be assigned to a pharmacist group with the lowest busyness degree for review.

In some embodiments, the busyness degree information comprises information indicating whether the pharmacist group is idle and a current processing capacity value of the pharmacist group. Thus, the prescription order can be assigned to an idle pharmacist group, or a pharmacist group with a greatest current processing capacity value, and the like.

In step S106, the prescription order is sent to a queue corresponding to the pharmacist group to review the prescription order. Thus, a pharmacist in the corresponding pharmacist group can obtain the prescription order from the queue and review the prescription order.

In some embodiments, prescription orders in a queue are evenly distributed to pharmacists in a pharmacist group based on the availability of the pharmacists. For example, after a pharmacist has processed the current prescription order, the pharmacist is in the idle state and is available to receive a newly distributed prescription order.

The queue has a first-in first-out feature, that is, a newly distributed prescription order is inserted into the tail of the queue, and a pharmacist reads and processes prescription an order in the queue from the head of the queue in sequence.

In some embodiments, a prescription order distributed to a pharmacist is sent to the pharmacist's processing terminal.

In step S108, a review result of the prescription order given by a pharmacist in the pharmacist group is acquired.

In the above embodiment, orders are distributed in units of pharmacist groups. After a prescription order is distributed to a queue, a pharmacist for processing the order can be further determined according to the situation of each pharmacist in the pharmacist group. Since the processing conditions of multiple pharmacists in the pharmacist group are comprehensively considered, the processing ability of individual pharmacists will not immoderately affect the overall processing ability of the pharmacist group, so that prescription orders can be processed timely. Therefore, the above embodiment can improve order processing efficiency in the scenario of distributing prescription orders. The above method is applicable even in high concurrency scenarios.

An embodiment of how to determine a pharmacist group to review a prescription order will be described below with reference to FIG. 2.

FIG. 2 shows a schematic flowchart of a method for determining a pharmacist group according to some embodiments of the present invention. As shown in FIG. 2, the method of determining a pharmacist group in this embodiment comprises steps S202 to S206.

In step S202, it is determined whether there is currently an idle pharmacist group.

In some embodiments, an idle pharmacist group is a pharmacist group having an idle value greater than a preset value, wherein the idle value is a difference between a sum of actual amount (i.e. numbers) of processing of all pharmacist(s) in the pharmacist group and a number of prescription order(s) in a queue corresponding to the pharmacist group.

In some embodiments, the actual amount of processing of each pharmacist is a product of a historical average amount of processing in unit time and an online time ratio of the pharmacist.

For example, suppose there are N pharmacists in a pharmacist group, and the ith pharmacist is represented as $P_i$, wherein $i \in (1, 2 \ldots N)$. The average time for the pharmacist group to process prescription orders is

$$T_i \triangleq \sum_{q \in Q_i}^{i} t_{i,q} / |Q_i|$$

, wherein the symbol "$\triangleq$" means "is defined as", and wherein $t_{i,q}$ is the time for the i-th pharmacist to process the prescription order q, $Q_i$ is a set of historical prescription orders reviewed by the i-th pharmacist, $|Q_i|$ represents the number of prescription orders in $Q_i$.

After $T_i$ is determined according to the historical records, the maximum amount of processing of the pharmacist in unit time can also be determined as

$$C_i \triangleq 1/T_i$$

. Considering that pharmacists responsible to review of prescription orders may be sometimes offline, the actual amount of processing of a pharmacist in unit time can be defined as

$$B_i \triangleq C_i(1 - A_i)$$

, wherein $A_i$ is the absence rate of the i-th pharmacist in unit time.

After obtaining the above information of the pharmacists in each pharmacist group, the idle value of the pharmacist group can be determined as

$$Y_j \triangleq \sum_{i \in n_j} B_i - U_j$$

, $j \in J$, wherein J is a set of pharmacist groups, and $n_j$ is a set of online pharmacists in the j-th pharmacist group, $U_j$ is the number of prescription orders in the queue corresponding to the j-th pharmacist group, that is, the number of prescription orders that have been distributed to the j-th pharmacist group and have not been processed yet. In the case that $Y_j$ is greater than 0, it can be considered that the j-th pharmacist group is idle, because, for this pharmacist group, the distributed prescription orders will not be overstocked for a long time.

By considering the situation where some pharmacists may be offline, and determining the idle value for a pharmacist group according to the actual amount of processing of online pharmacists, the prescription orders can be distributed more accurately, and the processing efficiency of the prescription orders can be improved.

In step S204, an idle pharmacist group is determined as the pharmacist group to review the prescription order in the case that there is currently an idle pharmacist group.

When distributing a prescription order, if there are multiple idle pharmacist groups, the prescription order can be distributed to the queue corresponding to a pharmacist group with a greatest idle value.

In step S206, a pharmacist group with a greatest current processing capacity value is determined as the pharmacist group to review the prescription order in case that there is currently no idle pharmacist group.

In some embodiments, the current processing capacity value is a difference between a sum of actual amount of processing of online pharmacist(s) in a pharmacist group and a number of prescription order(s) in a queue corresponding to the pharmacist group. The maximum amount of processing of a pharmacist group is

$$D_j \triangleq \sum_{i \in N_j} B_i$$

, $j \in J$, wherein $N_j$ is a set of all pharmacists in the j-th pharmacist group.

In some embodiments, when a prescription order is sent to the queue corresponding to the pharmacist group to review the prescription order, or when a pharmacist in the pharmacist group completes the review of the prescription order, the above information can be dynamically modified, so that subsequent prescription orders can be distributed more accurately.

Through the method of the above embodiment, the prescription order can be distributed to an idle pharmacist group as much as possible, and when there is no idle pharmacist group at present, the prescription order can be distributed according to the maximum processing capacity of the pharmacist groups. Therefore, the distributed prescription order can be processed faster, and thereby improving the efficiency of prescription order processing.

In some embodiments, the busyness degree information of pharmacist groups, the actual amount of processing of pharmacists, and the maximum current processing capacity value is stored in a database, and can be synchronized from the database to a cache and then stored in the cache. This database is regarded as, for example, a database for synchronization. In response to receiving a prescription order from a user terminal, busyness degree information of a plurality of pharmacist groups can be acquired from the cache. Therefore, the reading speed can be increased, and the processing efficiency of the prescription orders can be further improved.

In some embodiments, the busyness degree information in the cache has a data lock. Therefore, when multiple processing nodes perform operations of data reading or modification, it is possible to avoid the generation of dirty data, so that the acquired data is more accurate, and the processing efficiency is improved.

The busyness degree information can be updated when the distribution or review of a prescription order is completed. In some embodiments, the busyness degree information of the corresponding pharmacist group in the database is modified in response to the prescription order being sent to the queue corresponding to the pharmacist group to review the prescription order; and the busyness degree information of the corresponding pharmacist group in the database is modified, and the review result of the prescription order is stored into the database in response to completing the review of the prescription order by the pharmacist in the pharmacist group.

In some embodiments, the database is an HBase database. The HBase database is a columnar database that can be read at a faster speed. For example, the modified busyness degree information in the HBase database can be synchronized to a cache and a persistence database. The persistence database may comprise, for example, a MySQL or ORACLE database, etc.; and the review results of the prescription orders in the HBase database can be synchronized to a persistence database.

When all pharmacists in a pharmacist group are offline, the prescription orders already in the queue of that pharmacist group cannot be processed for a long period of time. The present invention also provides a disaster recovery solution to deal with this situation.

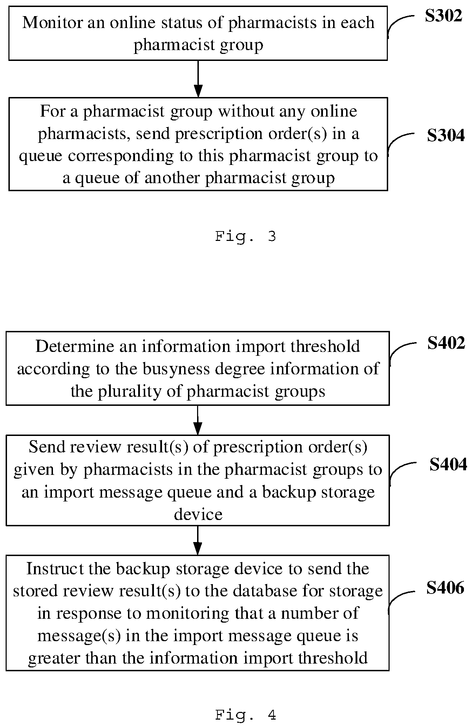

FIG. 3 shows a schematic flowchart of a disaster recovery method according to some embodiments of the present invention. As shown in FIG. 3, the disaster recovery method of this embodiment comprises steps S302 to S304.

In step S302, an online status of pharmacists in each pharmacist group is monitored.

In step S304, for a pharmacist group without any online pharmacists, prescription order(s) in a queue corresponding to this pharmacist group are sent to a queue of another pharmacist group.

In some embodiments, the another pharmacist group is a pharmacist group with a greatest current processing capacity value.

Therefore, it can be ensured that the orders can be processed in time, thereby improving the efficiency of prescription order processing.

The present invention also provides a message queue (MQ for short) compensation scheme to ensure that all prescription orders can be processed. An embodiment of the MQ compensation method will be described below with reference to FIG. 4.

FIG. 4 shows a schematic flowchart of the MQ compensation method according to some embodiments of the present invention. As shown in FIG. 4, the MQ compensation method of this embodiment comprises steps S402 to S406.

In step S402, an information import threshold is determined according to the busyness degree information of the plurality of pharmacist groups. The busyness degree information may be positively correlated with the information import threshold, for example, the information import threshold can be obtained by multiplying the absolute value of the idle value by a preset ratio. Since the busyness degree information changes dynamically, the information import threshold can also be dynamically determined.

In step S404, review result(s) of prescription order(s) given by pharmacists in the pharmacist groups are sent to an import message queue and a backup storage device, wherein the review result(s) in the import message queue are sequentially read and stored to the database.

In step S406, the backup storage device is instructed to send the stored review result(s) to the database for storage in response to monitoring that a number of message(s) in the import message queue is greater than the information import threshold.

Therefore, if the message queue is blocked, in order not to affect the processing of subsequent orders, the data can be stored by the backup storage device, which can improve the accuracy of data processing.

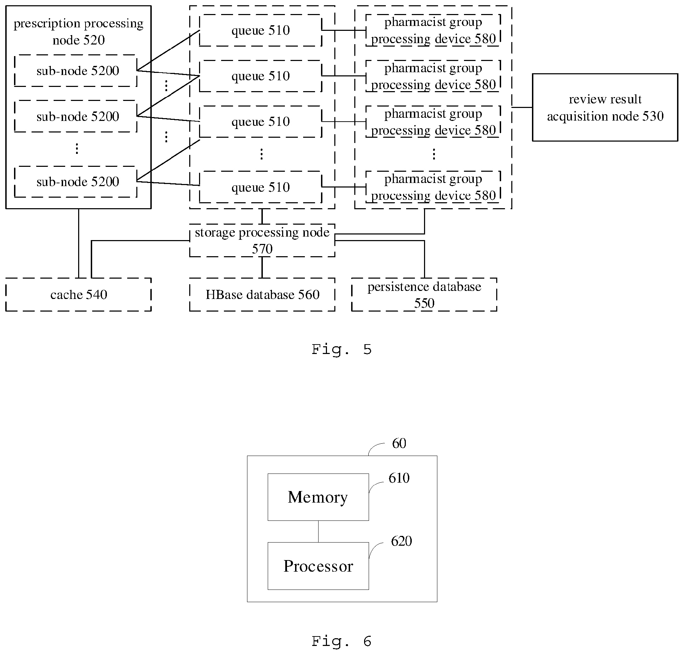

An embodiment of a distribution system of prescription order of the present invention will be described below with reference to FIG. 5.

FIG. 5 shows a schematic structural diagram of a distribution system of prescription order according to some embodiments of the present invention. As shown in FIG. 5, the distribution system 50 of prescription order of this embodiment comprises: a plurality of queues 510, each queue corresponding to a pharmacist group, and each pharmacist group comprising one or more pharmacists to review prescription orders in the corresponding queue; a prescription processing node 520 for acquiring busyness degree information of a plurality of pharmacist groups in response to receiving a prescription order from a user terminal; determining a pharmacist group to review the prescription order according to the busyness degree information; and sending the prescription order to a queue corresponding to the pharmacist group to review the prescription order; and a review result acquisition node 530 configured for acquiring a review result of the prescription order given by a pharmacist in the pharmacist group.

In some embodiments, the busyness degree information comprises information indicating whether a pharmacist group is idle and a current processing capacity value of the pharmacist group; and the prescription processing node 520 is further configured for determining an idle pharmacist group as the pharmacist group to review the prescription order in case that there is currently an idle pharmacist group; and determining a pharmacist group with a greatest current processing capacity value as the pharmacist group to review the prescription order in case that there is currently no idle pharmacist group.

In some embodiments, the idle pharmacist group is a pharmacist group having an idle value greater than a preset value, wherein the idle value is a difference between a sum of actual amount of processing of all pharmacist(s) in the pharmacist group and a number of prescription order(s) in a queue corresponding to the pharmacist group; and the current processing capacity value is a difference between a sum of actual amount of processing of online pharmacist(s) in a pharmacist group and a number of prescription order(s) in a queue corresponding to the pharmacist group.

In some embodiments, the actual amount of processing of each pharmacist is a product of a historical average amount of processing in unit time and an online time ratio of the pharmacist.

In some embodiments, the prescription processing node 520 is further configured for monitoring an online status of pharmacists in each pharmacist group; and for a pharmacist group without any online pharmacist, sending prescription order(s) in a queue corresponding to this pharmacist group to a queue of another pharmacist group.

In some embodiments, the another pharmacist group is a pharmacist group with a greatest current processing capacity value.

In some embodiments, the prescription processing node 520 comprises a plurality of distributed sub-nodes 5200.

In some embodiments, the system 50 for prescription order distribution further comprises: a cache 540 for storing busyness degree information of the plurality of pharmacist groups, wherein the busyness degree information in the cache is retrieved from a database and stored in the cache.

In some embodiments, the busyness degree information in the cache 540 has a data lock.

In some embodiments, the system 50 for prescription order distribution further comprises: a persistence database 550 for storing the busyness degree information and the review result of the prescription order; and an HBase database 560 for storing busyness degree information synchronized with the persistence database, wherein the busyness degree information in the HBase database is further synchronized to the cache.

In some embodiments, the distribution system 50 of prescription order further comprises: a storage processing node 570 configured for: modifying the busyness degree information of the corresponding pharmacist group in a database in response to the prescription order being sent to the queue corresponding to the pharmacist group to review the prescription order; and modifying the busyness degree information of the corresponding pharmacist group in the database, and storing the review result of the prescription order in the database in response to completing the review of the prescription order by the pharmacist in the pharmacist group.

In some embodiments, the storage processing node 570 is further configured for: determining an information import threshold according to the busyness degree information of the plurality of pharmacist groups; sending review result(s) of prescription order(s) given by pharmacists in the pharmacist groups to an import message queue and a backup storage device, wherein the review result(s) in the import message queue are sequentially read and stored to the database; and instructing the backup storage device to send the stored review result(s) to the database for storage in response to monitoring that a number of message(s) in the import message queue is greater than the information import threshold.

In some embodiments, the distribution system 50 of prescription order further comprises: a pharmacist group processing device 580 configured for reading a prescription order from a corresponding queue, and sending the prescription order to a terminal device of a pharmacist in the pharmacist group; and acquiring a review result after the pharmacist completes the review of the prescription order.

FIG. 6 shows a schematic structural diagram of a device for prescription order distribution according to some embodiments of the present invention. As shown in FIG. 6, the device 60 for prescription order distribution of this embodiment comprises: a memory 610 and a processor 620 coupled to the memory 610, the processor 620 configured to, based on instructions stored in the memory 610, carry out the method for prescription order distribution according to any one of the foregoing embodiments.

The memory 610 may comprise, for example, system memory, a fixed non-volatile storage medium, or the like. The system memory stores, for example, an operating system, application programs, a boot loader (Boot Loader), and other programs.

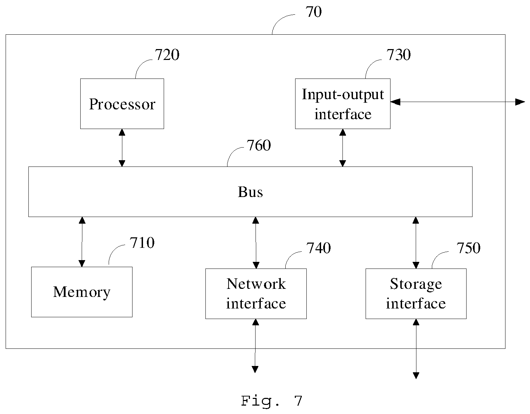

FIG. 7 shows a schematic structural diagram of a device for prescription order distribution according to other embodiments of the present invention. As shown in FIG. 7, the device 70 for prescription order distribution of this embodiment comprises: a memory 710 and a processor 720, and may further comprise an input-output interface 730, a network interface 740, a storage interface 750, and the like. These interfaces 730, 740, 750, the memory 710 and the processor 720 may be connected through a bus 760, for example. The input-output interface 730 provides a connection interface for input-output devices such as a display, a mouse, a keyboard, and a touch screen. The network interface 740 provides a connection interface for various networked devices. The storage interface 750 provides a connection interface for external storage devices such as an SD card and a USB flash disk.

An embodiment of the present invention also provides a computer-readable storage medium on which a computer program is stored, which is characterized in that when the program is executed by a processor, any one of the foregoing distribution methods of prescription order is implemented.

One skilled in the art should understand that, the embodiments of this invention may be provided as a method, a system, or a computer program product. Therefore, embodiments of this invention can take the form of an entirely hardware embodiment, an entirely software embodiment or an embodiment containing both hardware and software elements. Moreover, the present invention may take the form of a computer program product embodied on one or more computer-usable non-transitory storage media (including but not limited to disk storage, CD-ROM, optical memory, etc.) having computer-usable program code embodied therein.

The present invention has been described with reference to flow charts and/or block diagrams of methods, apparatuses (systems) and computer program products according to embodiments of the present invention. It should be understood that each process and/or block in the flowcharts and/or block diagrams, and combinations of the processes and/or blocks in the flowcharts and/or block diagrams may be implemented by computer program instructions. The computer program instructions may be provided to a processor of a general purpose computer, a special purpose computer, an embedded processor, or other programmable data processing apparatus to generate a machine such that the instructions executed by a processor of a computer or other programmable data processing apparatus to generate means implementing the functions specified in one or more flows of the flowcharts and/or one or more blocks of the block diagrams.

The computer program instructions may also be stored in a computer readable storage device capable of directing a computer or other programmable data processing apparatus to operate in a specific manner such that the instructions stored in the computer readable storage device produce an article of manufacture including instruction means implementing the functions specified in one or more flows of the flowcharts and/or one or more blocks of the block diagrams.

These computer program instructions can also be loaded onto a computer or other programmable device to perform a series of operation steps on the computer or other programmable device to generate a computer-implemented process such that the instructions executed on the computer or other programmable device provide steps implementing the functions specified in one or more flows of the flowcharts and/or one or more blocks of the block diagrams.

The above is merely preferred embodiments of this invention, and is not limitation to this invention. Within spirit and principles of this utility model, any modification, replacement, improvement and etc. shall be contained in the protection scope of this invention.

**Claims**

1. A distribution method of prescription order, comprising:

   acquiring busyness degree information of a plurality of pharmacist groups in response to receiving a prescription order from a user terminal, wherein each of the pharmacist groups corresponds to a queue and comprises pharmacist(s) to review prescription order(s) in the corresponding queue;
   determining a pharmacist group to review the prescription order according to the busyness degree information;
   sending the prescription order to a queue corresponding to the pharmacist group to review the prescription order; and
   acquiring a review result of the prescription order given by a pharmacist in the pharmacist group.

2. The distribution method according to claim 1, wherein acquiring busyness degree information of a plurality of pharmacist groups comprises:
   acquiring busyness degree information of a plurality of pharmacist groups from a cache in response to receiving a prescription order from the user terminal, wherein the busyness degree information in the cache is retrieved from a database and stored in the cache.

3. The distribution method according to claim 2, wherein the busyness degree information in the cache has a data lock.

4. The distribution method according to claim 2, further comprising:

   modifying the busyness degree information of the corresponding pharmacist group in the database in response to the prescription order being sent to the queue corresponding to the pharmacist group to review the prescription order; and
   modifying the busyness degree information of the corresponding pharmacist group in the database, and storing the review result of the prescription order in the database in response to completing the review of the prescription order by the pharmacist in the pharmacist group.

5. The distribution method according to claim 4, wherein the database is an HBase database, and the distribution method further comprises:

   synchronizing the modified busyness degree information in the HBase database to the cache and a persistence database; and
   synchronizing the review result of the prescription order in the HBase database to the persistence database.

6. The distribution method according to any one of claims 1 to 5, wherein the busyness degree information comprises information indicating whether a pharmacist group is idle and a current processing capacity value of the pharmacist group, and determining a pharmacist group to review the prescription order according to the busyness degree information comprises:

   determining an idle pharmacist group as the pharmacist group to review the prescription order in case that there is currently an idle pharmacist group; and
   determining a pharmacist group with a greatest current processing capacity value as the pharmacist group to review the prescription order in case that there is currently no idle pharmacist group.

7. The distribution method according to claim 6, wherein:

the idle pharmacist group is a pharmacist group having an idle value greater than a preset value, wherein the idle value is a difference between a sum of actual amount of processing of all pharmacist(s) in the pharmacist group and a number of prescription order(s) in a queue corresponding to the pharmacist group; and
the current processing capacity value is a difference between a sum of actual amount of processing of online pharmacist(s) in a pharmacist group and a number of prescription order(s) in a queue corresponding to the pharmacist group.

8. The distribution method according to claim 7, wherein the actual amount of processing of each pharmacist is a product of a historical average amount of processing in unit time and an online time ratio of the pharmacist.

9. The distribution method according to claim 1, further comprising:

monitoring an online status of pharmacists in each pharmacist group; and
for a pharmacist group without any online pharmacist, sending prescription order(s) in a queue corresponding to this pharmacist group to a queue of another pharmacist group.

10. The distribution method according to claim 9, wherein the another pharmacist group is a pharmacist group with a greatest current processing capacity value.

11. The distribution method according to claim 1, further comprising:

determining an information import threshold according to the busyness degree information of the plurality of pharmacist groups;
sending review result (s) of prescription order(s) given by pharmacists in the pharmacist groups to an import message queue and a backup storage device, wherein the review result(s) in the import message queue are sequentially read and stored to the database; and
instructing the backup storage device to send the stored review result(s) to the database for storage in response to monitoring that a number of message(s) in the import message queue is greater than the information import threshold.

12. A distribution system of prescription order, comprising:

a plurality of queues, each queue corresponding to a pharmacist group, and each pharmacist group comprising one or more pharmacists to review prescription orders in the corresponding queue;
a prescription processing node for acquiring busyness degree information of a plurality of pharmacist groups in response to receiving a prescription order from a user terminal; determining a pharmacist group to review the prescription order according to the busyness degree information; and sending the prescription order to a queue corresponding to the pharmacist group to review the prescription order; and
a review result acquisition node for acquiring a review result of the prescription order given by a pharmacist in the pharmacist group.

13. The distribution system according to claim 12, further comprising:
a cache for storing busyness degree information of the plurality of pharmacist groups, wherein the busyness degree information in the cache is retrieved from a database and stored in the cache.

14. The distribution system according to claim 13, wherein the busyness degree information in the cache has a data lock.

15. The distribution system according to claim 13, further comprising:

a persistence database for storing the busyness degree information and the review result of the prescription order; and
an HBase database for storing busyness degree information synchronized with the persistence database, wherein the busyness degree information in the HBase database is further synchronized to the cache.

16. The distribution system according to claim 12, further comprising:
a storage processing node for: modifying the busyness degree information of the corresponding pharmacist group in a database in response to the prescription order being sent to the queue corresponding to the pharmacist group to review the prescription order; and modifying the busyness degree information of the corresponding pharmacist group in the database, and storing the review result of the prescription order in the database in response to completing the review of the prescription order by the pharmacist in the pharmacist group.

**EP 4 160 608 A1**

**17.** The distribution system according to claim 16, wherein the storage processing node is further configured for: determining an information import threshold according to the busyness degree information of the plurality of pharmacist groups; sending review result(s) of prescription order(s) given by pharmacists in the pharmacist groups to an import message queue and a backup storage device, wherein the review result(s) in the import message queue are sequentially read and stored to the database; and instructing the backup storage device to send the stored review result(s) to the database for storage in response to monitoring that a number of message(s) in the import message queue is greater than the information import threshold.

**18.** The distribution system according to any one of claims 12 to 17, wherein the prescription processing node comprises a plurality of distributed sub-nodes.

**19.** A prescription order distribution device, comprising:

a memory; and
a processor coupled to the memory, the processor configured to, based on instructions stored in the memory, carry out the distribution method of prescription order according to any one of claims 1 to 11.

**20.** A computer-readable storage medium on which a computer program is stored, which when executed by a processor implements the distribution method of prescription order according to any one of claims 1 to 11.

Acquire busyness degree information of a plurality of pharmacist groups in response to receiving a prescription order from a user terminal — S102

Determine a pharmacist group to review the prescription order according to the busyness degree information — S104

Send the prescription order to a queue corresponding to the pharmacist group to review the prescription order — S106

Acquire a review result of the prescription order given by a pharmacist in the pharmacist group — S108

FIG. 1

Determine whether there is currently an idle pharmacist group — S202

Yes

No

Determine an idle pharmacist group as the pharmacist group to review the prescription order — S204

Determine a pharmacist group with a greatest current processing capacity value as the pharmacist group to review the prescription order — S206

Fig. 2

EP 4 160 608 A1

Monitor an online status of pharmacists in each pharmacist group — S302

For a pharmacist group without any online pharmacists, send prescription order(s) in a queue corresponding to this pharmacist group to a queue of another pharmacist group — S304

Fig. 3

Determine an information import threshold according to the busyness degree information of the plurality of pharmacist groups — S402

Send review result(s) of prescription order(s) given by pharmacists in the pharmacist groups to an import message queue and a backup storage device — S404

Instruct the backup storage device to send the stored review result(s) to the database for storage in response to monitoring that a number of message(s) in the import message queue is greater than the information import threshold — S406

Fig. 4

prescription processing node 520

sub-node 5200

sub-node 5200

sub-node 5200

queue 510

queue 510

queue 510

queue 510

pharmacist group processing device 580

pharmacist group processing device 580

pharmacist group processing device 580

pharmacist group processing device 580

review result acquisition node 530

storage processing node 570

cache 540

HBase database 560

persistence database 550

Fig. 5

60

Memory 610

Processor 620

Fig. 6

70

720

Processor

730

Input-output
interface

760

Bus

710

Memory

740

Network
interface

750

Storage
interface

Fig. 7

<div align="center">

**INTERNATIONAL SEARCH REPORT**

</div>

| | International application No. |
|---|---|
| | **PCT/CN2021/091490** |

| A. | CLASSIFICATION OF SUBJECT MATTER |
|---|---|
| | G16H 20/13(2018.01)i |

According to International Patent Classification (IPC) or to both national classification and IPC

| B. | FIELDS SEARCHED |
|---|---|

Minimum documentation searched (classification system followed by classification symbols)
　　G16H

Documentation searched other than minimum documentation to the extent that such documents are included in the fields searched

Electronic data base consulted during the international search (name of data base and, where practicable, search terms used)
　　WPI, EPODOC, CNPAT, CNKI, IEEE: 组, 队, 分配, 分发, 分派, 派发, 订单, 定单, 忙, 闲, 药师, 药剂师, 处方, pharmacist, prescription, group, team, distribute, order, busy, free

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 111785346 A (BEIJING WODONG TIANJUN INFORMATION TECHNOLOGY CO., LTD. et al.) 16 October 2020 (2020-10-16)<br>　　claims 1-20 | 1-20 |
| Y | CN 110706775 A (BEIJING PHARMA CONSCIENCE INFORMATION TECHNOLOGY CO., LTD.) 17 January 2020 (2020-01-17)<br>　　description, paragraphs [0006]-[0008] and [0107]-[0110] | 1-20 |
| Y | CN 107222646 A (HUAWEI TECHNOLOGIES CO., LTD.) 29 September 2017 (2017-09-29)<br>　　description, paragraphs [0009]-[0039] | 1-20 |
| A | CN 105224794 A (SHI, Qingping) 06 January 2016 (2016-01-06)<br>　　entire document | 1-20 |
| A | CN 107403295 A (BEIJING XIAODU INFORMATION TECHNOLOGY CO., LTD.) 28 November 2017 (2017-11-28)<br>　　entire document | 1-20 |
| A | CN 110706798 A (WUHAN ANQUANJIA INFORMATION TECHNOLOGY CO., LTD.) 17 January 2020 (2020-01-17)<br>　　entire document | 1-20 |

☑ Further documents are listed in the continuation of Box C. 　　　☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 July 2021** | **29 July 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/091490**

**C. DOCUMENTS CONSIDERED TO BE RELEVANT**

| Category* | Citation of document, with indication, where appropriate, of the relevant passages | Relevant to claim No. |
|---|---|---|
| A | CN 110634555 A (YINCHUAN YOULAI INTERNET HOSPITAL CO., LTD.) 31 December 2019 (2019-12-31) entire document | 1-20 |
| A | CN 103533068 A (LI, Liang) 22 January 2014 (2014-01-22) entire document | 1-20 |

Form PCT/ISA/210 (second sheet) (January 2015)

INTERNATIONAL SEARCH REPORT
Information on patent family members

International application No.

**PCT/CN2021/091490**

| Patent document cited in search report | | | Publication date (day/month/year) | Patent family member(s) | | | Publication date (day/month/year) |
|---|---|---|---|---|---|---|---|
| CN | 111785346 | A | 16 October 2020 | None | | | |
| CN | 110706775 | A | 17 January 2020 | None | | | |
| CN | 107222646 | A | 29 September 2017 | None | | | |
| CN | 105224794 | A | 06 January 2016 | None | | | |
| CN | 107403295 | A | 28 November 2017 | WO | 2019000780 | A1 | 03 January 2019 |
| | | | | CN | 111950959 | A | 17 November 2020 |
| CN | 110706798 | A | 17 January 2020 | None | | | |
| CN | 110634555 | A | 31 December 2019 | None | | | |
| CN | 103533068 | A | 22 January 2014 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010479267